(19) **Europäisches Patentamt**
**European Patent Office**
**Office européen des brevets**

(11) **EP 2 591 854 A1**

(12) **EUROPEAN PATENT APPLICATION**
published in accordance with Art. 153(4) EPC

(43) Date of publication:
**15.05.2013 Bulletin 2013/20**

(51) Int Cl.:
*B01J 23/30* (2006.01)      *B01J 23/20* (2006.01)
*C07C 45/38* (2006.01)      *C07C 47/22* (2006.01)
*C07B 61/00* (2006.01)      *C07C 27/00* (2006.01)
*C07C 51/235* (2006.01)      *C07C 57/04* (2006.01)

(21) Application number: **11803682.1**

(22) Date of filing: **08.07.2011**

(86) International application number:
**PCT/JP2011/065669**

(87) International publication number:
**WO 2012/005348 (12.01.2012 Gazette 2012/02)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **09.07.2010 JP 2010156783**

(71) Applicants:
• **Nippon Kayaku Kabushiki Kaisha**
  **Tokyo 102-8172 (JP)**
• **National University Corporation Hokkaido University**
  **Sapporo-shi, Hokkaido 060-0808 (JP)**

(72) Inventors:
• **UEDA, Wataru**
  **Sapporo-shi, Hokkaido 060-0808 (JP)**
• **MAGATANI, Yasuhiro**
  **Tokyo 102-8172 (JP)**
• **OKUMURA, Kimito**
  **SanyoOnoda-shi, Yamaguchi 757-8686 (JP)**
• **KAWAGUCHI, Toru**
  **SanyoOnoda-shi, Yamaguchi 757-8686 (JP)**

(74) Representative: **Gille Hrabal**
  **Brucknerstrasse 20**
  **40593 Düsseldorf (DE)**

(54) **NOVEL GLYCEROL DEHYDRATION CATALYST AND PRODUCTION METHOD THEREFOR**

(57)      Provided is a novel catalyst that can produce acrolein and acrylic acid in high yields using glycerol as starting material. The disclosed glycerol dehydration catalyst has niobic oxide synthesized by hydrothermal synthesis as the main component.

EP 2 591 854 A1

**Description**

**Technical Field**

This invention relates to a novel dehydration catalyst. In particular, this invention relates to a novel catalyst used in catalytic dehydration reaction of glycerin in gas phase of liquid phase to produce acrolein and acrylic acid, and to a method for preparing the catalyst.

**Background Art**

Today's industrial production process for preparing unsaturated aldehyde and unsaturated carboxylic acids such as acrolein and acrylic acid is gas-phase catalytic oxidation of propylene which is used as a starting material. However, from the viewpoint of global warming and petroleum depletion, it is requested recently to develop another technique to produce fuels and organic products from bio resources that do not depend on fossil resources. An example of such products is bio-ethanol and bio-diesel obtained by a conversion of fats and oils, the output of which may exceed 20 million ton/year. Since they are produced from vegetable oil, that they can be an alternative fuel of fossil fuel and decrease discharge of carbon dioxide, so that their increase in demand is expected. In a process for preparing the bio-diesel, such a large amount as about 1/10 of the total output of glycerin is produced as a byproduct. Part of the glycerin produced is utilized as materials for medicines, cosmetic and foodstuff additives but a large amount of the glycerin is disposed in a form of industrial waste due to in balance between demand and supply. Therefore, new synthesis reactions are researched very actively to develop novel uses of glycerin and/or to expand its demand.

A variety of catalysts were developed in the industrial process to produce acrolein and acrylic acid by the dehydration of glycerin and we also filed many patent applications. For example, Patent Document 1 (JP-A1-2010-99596) discloses a glycerin dehydration catalyst of phosphorus-vanadium type compound oxide or its precursor which contains phosphorus and vanadium as indispensable constituent elements, used in production of acrolein and acrylic acid by catalytic dehydration reaction of glycerin.

Patent Document 2 (JP-A1-2006-290815) discloses a process for producing acrolein by a liquid-phase dehydration reaction of glycerin which was dispersed in a solvent in the presence of an acid solid catalyst whose $H_0$ is from -5.6 to +3.3 such as $KHSO_4$ and $K_2SO_4$. This method, however, produces more than 10% of carbides and hence can't be used in actual industry and there is a room of improvement.

In the production of acrolein by the liquid-phase or gas-phase catalytic reaction of glycerin, a solid acid catalyst having the $H_0$ value of lower than +2 is effective. For example, Patent Document 3 (JP-A1-6-211724) teaches $\alpha$-$Al_2O_3$ catalyst carrying phosphoric acid. In this patent, the yield of acrolein is 75% in gas-phases at 300°C. However, there is a problem in the catalyst life and generation of by-products such as hydroxyacetone increase in time, so that the selectivity is lowered.

In Patent Document 4 (WO2006/087083) and Patent Document 5 (WO2006/087084), oxygen is introduced in the in gas-phase glycerin dehydration reaction to prevent the of degradation of catalyst. In these patents, the catalyst having the acid strength of $H_0$ of higher than -9 but lower than -18 is used.

Non-Patent Document 1 (S.H.Chai et al. Journal of Catalysis 250 (2007) 342-349) discloses that acrolein was produced by gas-phase dehydration reaction of glycerol by using a catalyst which was prepared by a calcination of water-containing niobic acid. However, the highest yield is merely 45%. Therefore, this yield of acrolein obtained by using the niobium oxide can't be used in actual industrial plant.

**Prior arts**

**Patent Document**

Patent Document 1:     JP-A1-2010-99596
Patent Document 2:     JP-A1-2006-290815
Patent Document 3:     JP-A1-6-211724
Patent Document 4:     WO2006/087083
Patent Document 5:     WO2006/087084

**Non-Patent Document**

Non-Patent Document 1: S.H.Chai et al. Journal of Catalysis 250 (2007) 342-349

**Disclosure of Invention**

**Technical Problems**

An object of this invention is to provide a novel catalyst which can produce acrolein and acrylic acid from glycerin which is a raw material which does not depend on fossil resources.
An object of this invention is to provide a novel catalyst which can produce acrolein and acrylic acid from glycerin as raw material at high yield.
An object of this invention is to provide a glycerin dehydration reaction catalyst which can produce acrolein and acrylic acid from glycerin as raw material at high yield.
An object of this invention is to provide a method for preparing the superscription catalyst.

**Technical Solution**

Inventors tried to solve the problem and found that acrolein and acrylic acid can be prepared at high yield by the dehydration reaction of glycerin by using a niobium oxide prepared by the hydrothermal synthesis technique and completed this invention.
The subjects of this invention was following (1) to (7) or their combinations:

(1) A catalyst used in a production of acrolein and acrylic acid by dehydration reaction of glycerin, wherein said catalyst comprises niobium oxide ($NbO_x$, in which Nb is niobium and x is any number) prepared by hydrothermal synthesis technique.
(2) Glycerin dehydration catalyst used in a production of acrolein and acrylic acid by dehydration reaction of glycerin, characterized in that a catalytic active component is a niobium oxide, wherein the niobium oxide ($NbO_x$),has each one diffraction peak at $2\theta=22.6\pm0.3°$ and $2\theta= 46.1\pm0.3°$ respectively in the X-ray diffraction pattern (Cu-K$\alpha$, $\lambda$ = 1.5418 nm).
(3) The niobium oxide contains further tungsten and has following formula: $W_bNbOx$ in which Nb is niobium, b >0, x is a value determined by the oxidation numbers of Nb and W.
(4) The catalyst represented by the following general formula (1):

$$A_a (W_bNbO_x) \cdot nH_2O \qquad (1)$$

in which A is at least one member selected from elements belonging to Group 1 to Group 16 of the Periodic Table of Elements and ammonium, W is tungsten, Nb is niobium, a $\leq$ 0, b $\leq$ 0, x is a number determined by oxidation numbers of the elements, and n is a any positive number.
(5) The catalyst in which the niobium oxide is added with at least one salt of elements belonging to Group 1 to Group 16 of the Periodic Table of Elements and ammonium.
(6) The catalyst is prepared by oxidizing a niobium compound which is changed to an oxide under heat by hydrothermal synthesis technique.
(7) Use of the catalyst in a process for preparing acrolein and acrylic acid by catalytic dehydration of glycerin.

**Advantageous Effect**

The process for produces acrolein and acrylic acid by the catalytic dehydration reaction of glycerin according to the present invention permits to realize higher yield of acrolein and acrylic acid and to improve the production efficiency, so that it is very advantageous in industrial uses. In fact, acrolein can be produced at high yield by using a niobium compound prepared by hydrothermal synthesis technique according to the present invention, while such sufficiently higher yield can't be obtained in case of a usual niobium compound which becomes an oxide by heating.

**Brief Description of drawings**

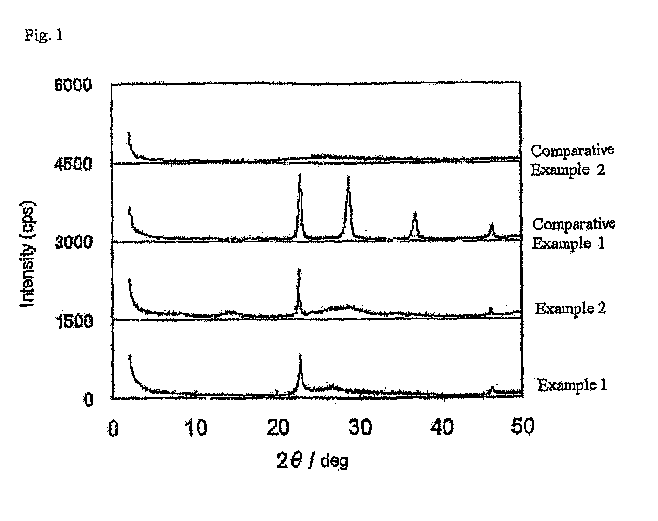

Fig. 1 shows the X-ray diffraction patterns of catalysts prepared in Examples.

**Mode for Carrying out the Invention**

The glycerin dehydration catalyst according to this invention is used to produce acrolein and acrylic acid by dehydrating glycerin and consists mainly of a niobium compound prepared by hydrothermal synthesis.

A raw material of the niobium compound is not limited specially if it can be used in hydrothermal synthesis technique as raw material and may be niobium oxide (II), niobium oxide (IV), niobium oxide (V), niobium hydroxide, niobium hydrogen oxalate, niobium chloride, niobium ethoxide, niobium butoxide and niobium phenoxide.

The condition of the hydrothermal synthesis is not limited specially. The hydrothermal synthesis can be carried out generally by keeping a temperature of 50 to 200°C, preferably from 70 to 170°C for a duration of from 1 to 300 hours. The reaction medium can be stirred during the hydrothermal synthesis. The hydrothermal synthesis reaction can be effected generally in an autoclave. An atmosphere in the autoclave also is not limited specially. When the hydrothermal synthesis complete, a reaction liquid is cooled, a solid content is filtered, washed and dried.

The niobium compound according to this invention is characterized in that it has diffraction peaks only at $2\theta = 22.6° \pm 0.3°$ and $46.1° \pm 0.3°$ respectively in its X-ray diffraction pattern (Cu-K$\alpha$ line, $\lambda$ = 1.5418 nm).

Above fact that its X-ray diffraction pattern (Cu-K$\alpha$ line $\lambda$ = 1.5418 nm) of niobium compound has the peaks only at $2\theta = 22.6° \pm 0.3°$ and $46.1° \pm 0.3°$ respectively means or reveals that (001) plane and (002) planes of TT-phase of niobium pentoxide grow during the hydrothermal synthesis process. The TT-phase of niobium pentoxide is here a crystal having a unit structure of $Nb_2O_5$ having a pseudo hexagonal structure ( see non-Patent Document 1).

The catalyst of this invention may be a niobium oxide containing further W (tungsten) in addition to the niobium oxide. The yield of acrolein is largely increased when tungsten is added to niobium oxide.

In the most preferable embodiment of this invention, the glycerin dehydration catalyst contains a compound such as compound oxide represented by the following general formula (1):

$$A_a (W_b NbO_x) \cdot n H_2O \qquad (1)$$

in which A is at least one member selected from elements belonging to Group 1 to Group 16 of the Periodic Table of Elements and ammonium, W is tungsten, Nb is niobium, $a \leq 0$, $b \leq 0$, x is a number determined by oxidation numbers of the elements, and n is a any positive number.

The catalyst according to this invention may be niobium oxides added with at least one compound of element chosen from the Group 1 to the Group 16 of the periodic table of elements in addition to the above niobium oxide. Niobium oxide obtained by such "addition" are those ion-exchanged, those added with a salt without ion-exchange and their mixtures.

The compound of element chosen from the Group 1 to the Group 16 of the periodic table of elements may be metal salt and onium salt. The metal salt may be salts of sodium, potassium, rubidium, cesium, magnesium, calcium, strontium, barium, scandium, yttrium, lanthanide, titanium, zirconium, hafnium, chromium, manganese, rhenium, iron, ruthenium, osmium, cobalt, palladium, copper, silver, gold, zinc, gallium, thallium, germanium, tin, lead, bismuth, tellurium, palladium, cerium, aluminum. The onium salt may be amine salt, ammonium salt, phosphonium salt and sulfonium salt. Raw materials of such metal salt or onium salt may be nitrate, carbonate, sulfates, acetates, oxides, halide and hydroxides of metals or of onium but are not limited to them. An amount of addition of the metal salt or metal or onium salt to the niobium oxide is 0.01 to 60 % by weight, preferably 0.01 to 30 % by weight.

The niobium oxide itself can be used as catalyst or the niobium oxide is preferably calcinated and then is used as catalyst. Person skilled in the arts can easily select the calcination conditions according to the nature of crystals.

The "hydrothermal synthesis" is often defined as a reaction in which water is involved at high temperatures and high pressure, generally higher than 100°C and higher than 1 atm. However, temperatures and pressure can be changed continuously, so that the "hydrothermal synthesis" can be cover all reactions in which water participate, including reactions effected under ambient temperature. The "hydrothermal synthesis" has such a merit that stable chemical compounds possessing high crystallinity can be realized, and hence is studied in many fields such as piezoelectric material, nano-sheet, photocatalyst, hydroxyapatite and ultrastructure.

In the present invention, a specific niobium oxide prepared by hydrothermal synthesis technique is used to produce acrolein and acrylic acid by the catalytic dehydration of glycerin. As far as inventors knows, such process was not known.

Patent Document 6 (WO2007/058221) discloses a method for producing acrolein by dehydration of glycerin in gas-phase in the presence of metallo silicate as a solid acid catalyst, obtained by hydrothermal synthesis. However, there is neither mention nor description of use of niobium oxide.
Patent Document 6 WO2007/058221

Patent Document 7 (Japanese patent No. 3,845,720) discloses a method for preparing a photo-catalyst of potassium niobate. In this patent, niobic acid is mixed with an aqueous solution of potassium hydroxide prepared by hydrothermal synthesis technique and supporting nickel ions. The X-ray diffraction diagram of a product prepared by this method shows diffraction peaks of potassium hexaniobate ($K_4Nb_6O_{17}$) and of potassium niobate ($KnbO_3$).
Patent Document 7 Japanese patent No. 3,845,720

In the glycerin dehydration catalyst for producing acrolein and acrylic acid according to this invention, the niobium oxide can be supported on a carrier. The carrier can be silica, diatomaceous earth, alumina, silica alumina, silica magnesia, zirconia, titania, magnesia, zeolite, silicon carbide and carbide. The catalyst can be supported on one of these carriers or on a complex or mixture of these carriers. Active substance can be utilized effectively by using the carrier. An amount of the niobium oxide on the support is usually 5 to 200 % by weight, preferably 10 to 150 % by weight.

The catalyst can have any shape and there is no limitation in their shape. The catalyst can be granule and powder or can be molded with the carrier and other auxiliary component to a shape of sphere, pellet, cylindrical body, hollow cylindical body by using a molding aid if necessity. The size of molded catalyst is suitably 1 to 10 mm for a fixed bed catalyst and the particle size less than 1 mm is for a fluidized bed catalyst.

The niobium oxide catalyst used in glycerin dehydration can be used after calcination and drying under reduced pressure which is effected as a pretreatment. Or, the niobium oxide catalyst can be used without such pretreatment. The calcination or firing can be carried out in air, nitrogen, reducing gas such as hydrogen, inert gas such as helium, argon or under a mixed gas atmosphere such as inert gas plus oxygen. The calcination can be carried out in any furnace such as muffle furnace, rotary kiln and fluidized bed furnace. There is no limitation thereof. Or, the calcination can be carried out in a reaction tube used for the glycerin dehydration reaction. The firing temperature is usual 150 to 700°C, preferably 200 to 600°C and more preferably 200 to 500°C. The calcination can be effected preferably for 0.5 to 30 hours.

The dehydration reaction of glycerin according to this invention can be effected in gas-phase or in liquid-phase but is carried out preferably in gas-phase. The gas-phase reaction can be carried out in a variety of reactors such as fixed bed, fluidized bed, circulating fluidized bed and movable bed. Among them a fixed bed is preferable.

Regeneration of catalyst can be effected outside the reactor. When the catalyst is taken out of a reactor system for regeneration, the catalyst is burnt in air or in oxygen-containing gas. In case of liquid phase reaction, usual general reactors for liquid reactions for solid catalysts can be used. Since the difference in boiling point between glycerin (290°C) and acrolein and acrylic acid is big, the reaction is effected preferably at relatively lower temperatures so as to distil out acrolein continuously.

The reaction temperature for producing acrolein and acrylic acid by dehydration of glycerin in gas-phase is effected preferably at a temperature of 200°C to 450°C. If the temperature is lower than 200°C, the life of catalyst will be shortened due to polymerization and carbonization of glycerin and of reaction products, because the boiling point of glycerin is high. On the contrary, if the temperature exceeds 450°C, the selectivity of acrolein and acrylic acid will be lowered due to increment in parallel reactions and successive reactions. Therefore, more preferable reaction temperature is 250°C to 350°C. The pressure is not limited specially but is preferably lower than 5 atm and more preferably lower than 3 atm. Under higher pressures, gasified glycerin will be re-liquefied and deposition of carbon will be promoted by higher pressure so that the life of catalyst will be shortened.

A feed rate of a material gas is preferably 500 to $10,000h^{-1}$ in term of the space velocity of GHSV. The selectivity will be lowered if the GHSV becomes lower than $500h^{-1}$ due to successive reactions. On the contrary, if the GHSV exceeds $10,000h^{-1}$, the conversion will be lowered.

In a case of liquid-phase reaction, the reaction temperature is preferably from 150°C to 350°C. The selectivity will be spoiled under lower temperatures although the conversion is improved. The reaction pressure is not limited specially but the reaction can be carried if necessary under a pressurized conditions of 3 atm to 70 atm.

The material of glycerin is easily available in a form of aqueous solution of glycerin. Concentration of the aqueous solution of glycerin is preferably from 5 % to 90 % by weight and more preferably 10 % to 50 % by weight. Too high concentration of glycerin will result in such problems as production of glycerin ethers or undesirable reaction between the resulting acrolein or acrylic acid and material glycerin, and temperature which is necessary to gasify glycerin is increased.

Now, the present invention will be explained in much detail with referring several examples, but this invention should not be limited to those described in following examples. In the following Examples and Comparative Examples, % means mole %.

### Examples

Example 1 (Preparation of NbOx)

7.4529 g of niobium hydrogen oxalate ($Nb(HC_2O_4)_5 \cdot nH_2O$) was dispersed in 25 ml of distilled water and was subjected to ultrasonic wave vibration for 10 minutes. The resulting dispersion was introduced in an autoclave and was subjected to hydrothermal synthesis at 175°C for three days. The resulting slurry was vacuum filtered, washed with distilled water and then dried at 80°C for one night to obtain a catalyst powder of NbOx. Fig. 1 shows X-ray diffraction pattern of a catalyst obtained. Fig. 1 shows each one peak at $2\theta=22.6$ and $2\theta=46.1$.

Evaluation of the reactivity of catalyst

The reactivity of catalyst obtained was evaluated in a fixed bed operated at ambient pressure through which reactants pass. The catalyst powder was shaped in a press and passed through a sieve to obtain particles having particle sizes of 9 to 12 meshes. 10 cc of the particles were packed in a reaction tube made of SUS (diameter of 20 mm) to form a catalyst bed. An aqueous solution of glycerin (concentration of 30 % by weight) was fed to an evaporator heated at 300°C at a flow rate of 21 g/hr by a pump, so that glycerin was gasified. The resulting gasified glycerin was passed through the fixed catalyst bed together with nitrogen. The fixed catalyst bed was heated at a temperature of 300°C. Feed gas had a following composition in mol %: glycerin : nitrogen: water = 6.3 : 18.9 : 74.8. GHSV was 2445 h$^{-1}$.

Products were condensed in a condenser and quantitative-analyzed by a gas chromatograph (GC-4000, GL Science, DB-WAX column). Proportions of products were corrected in factors from the results of the gas chromatograph to determine absolute amounts of products to calculate the conversion (%) of material (the conversion of glycerin) and the yield of objective substance (the yield of acrolein) from an amount of glycerin fed, an amount of glycerin remained and amounts of the products by following equations:

$$\text{The conversion (\%) of material} = (\text{a mole number of material reacted} \;/\; \text{a mole number of material supplied}) \times 100$$

$$\text{The yield (\%) of objective substance} = (\text{a mole number of objective substance obtained} \;/\; \text{a mole number of material fed}) \times 100$$

Results are summarized in Table 1.

Example 2 (reparation of $W_{0.5} Nb_{1.0} Ox$)

1.1376 g of niobium oxide ($Nb_2O_5 \cdot nH_2O$) was dispersed in 25 ml of distilled water and was subjected to ultrasonic wave vibration for10 minutes to prepare a dispersion of niobium oxide. In a separated flask, 0.7660 g of ammonium tungstophosphrate (($NH_4)_6[H_2W_{12}O_{40}] nH_2O$ was dissolved in 20 ml of distilled water. The resulting aqueous solution of ammonium tungstophosphrate was added into the dispersion of niobium oxide slowly. The resulting mixture was introduced in an autoclave and was subjected to hydrothermal synthesis at 175°C for three days. The resulting slurry was vacuum filtered, washed with distilled water and then dried at 80°C for one night to obtain a catalyst powder of NbOx. This catalyst shows X-ray diffraction pattern of Fig. 1 which has each one peak at $2\theta=22.6$ and $2\theta= 46.1$. This confirm that the resulting crystalline structure is similar to NbOx.

Evaluation of the reactivity of catalyst

The reactivity of the catalyst obtained was evaluated by the same method as Example 1 and the results are summarized in Table 1.

Example 3 (Preparation of Cs-exchanged $W_{0.5} Nb_{1.0} Ox$)

Ion-exchanged of Cs was effected as following: 0.2612 g of CsCl was added to 25 ml of distilled water. Into the resulting aqueous solution of CsCl, 1.0 g of WNbOx obtained in Example 2 was added and stirred at ambient temperature for 24 hours. Then, the resulting slurry was vacuum-filtered, washed with distilled water and then dried at 80°C for one night to obtain a Cs-exchanged WnbOx.

Evaluation of the reactivity of catalyst

The reactivity of the catalyst obtained was evaluated by the same method as Example 1 and the results are summarized in Table 1.

Example 4 (Calcinated sample of $W_{0.5} Nb_{1.0} Ox$)

Calcination of WNbOx was effected as following: a powder obtained in Example 2 was calcinated in a horizontal type electric furnace at 500°C in air for 3 hours to obtain a fired catalyst.

Evaluation of the reactivity of catalyst

The reactivity of the catalyst obtained was evaluated by the same method as Example 1 and the results are summarized in Table 1.

Comparative Example 1

Commercially available niobium oxide ($Nb_2O_5 \cdot nH_2O$, product of Mitsui Metal Co., Ltd.) was calcinated in air in a Muffle furnace at 500°C in air for 3 hours.
X-ray diffraction pattern of the resulting catalyst is shown in Fig. 1 in which strong peaks are observed at $2\theta$=22.8, 28.7, 36.9 and 46.4°, so that it was confirmed that they are typical peaks of niobium pentaoxide.

Evaluation of the reactivity of catalyst

The reactivity of the catalyst obtained was evaluated by the same method as Example 1 and the results are summarized in Table 1.

Comparative Example 2

Commercially available hydrogen oxalate ($Nb(HC_2O_4)_5 \cdot nH_2O$, SOEKAWA Chemical Co., Ltd.) was calcinated in air in a Muffle furnace at 500°C in air for 3 hours. The resulting catalyst shows no X-ray diffraction pattern as is shown in Fig. 1, so that it was confirmed that the catalyst is amorphous.

Evaluation of the reactivity of catalyst

The reactivity of the catalyst obtained was evaluated by the same method as Example 1 and the results are summarized in Table 1.

Table 1

|  | Glycerin conversion (%) | Acrolein yield (%) |
|---|---|---|
| Example 1 | 94.7 | 35.2 |
| Example 2 | 99.4 | 70.4 |
| Example 3 | 99.5 | 61.9 |
| Example 4 | 100.0 | 60.2 |
| Comparative 1 | 26.5 | 16.2 |
| Comparative 2 | 41.3 | 21.9 |

From the results of Examples and Comparative Examples, following conclusion was obtained:

(1) Such very high yield of acrolein as 70% was realized when a niobium oxide (NbOx), especially W-containing niobium oxide (WNbOx) which was prepared by hydrothermal synthesis technique according to the present invention was used in a production of acrolein and acrylic acid by the dehydration reaction of glycerin.

(2) On the other hand, the acrolein yield is only 16% in case of niobium pentoxide which is outside the present invention.

**Claims**

1.   A catalyst used in a production of acrolein and acrylic acid by dehydration reaction of glycerin, *characterized by* said catalyst comprises niobium oxide ($NbO_x$, in which Nb is niobium and x is any number) prepared by hydrothermal synthesis technique.

**2.** The catalyst of claim 1, wherein said niobium oxide ($NbO_x$) has each one diffraction peak at $2\theta=22.6\pm0.3°$ and $2\theta=46.1\pm0.3°$ respectively in the X-ray diffraction pattern (Cu-K$\alpha$, $\lambda$ = 1.5418 nm).

**3.** The catalyst of claim 1 or 2, wherein the said niobium oxide contains further tungsten and has following formula: $W_bNbOx$ in which Nb is niobium, b >0, x is a value determined by the oxidation numbers of Nb and W.

**4.** The catalyst of any one of claims 1 to 3, represented by the following general formula (1):

$$A_a (W_bNbO_x) \cdot nH_2O \quad (1)$$

in which A is at least one member selected from elements belonging to Group 1 to Group 16 of the Periodic Table of Elements and ammonium, W is tungsten, Nb is niobium, a $\leq$ 0, b $\leq$ 0, x is a number determined by oxidation numbers of the elements, and n is a any positive number.

**5.** The catalyst of any one of claims 1 to 4, wherein said niobium oxide is added with at least one salt of elements belonging to Group 1 to Group 16 of the Periodic Table of Elements and ammonium.

**6.** A method for preparing a catalyst used in a production of acrolein and acrylic acid by dehydration reaction of glycerin, *characterized in that* said catalyst is prepared by oxidizing a niobium compound which is changed to an oxide under heat by hydrothermal synthesis technique.

**7.** Use of the catalyst according to any one of claims 1 to 5 in a process for preparing acrolein and acrylic acid by catalytic dehydration of glycerin.

Fig. 1

| INTERNATIONAL SEARCH REPORT | International application No. |
|---|---|
| | PCT/JP2011/065669 |

A. CLASSIFICATION OF SUBJECT MATTER
*B01J23/30*(2006.01)i, *B01J23/20*(2006.01)i, *C07C45/38*(2006.01)i, *C07C47/22* (2006.01)i, *C07B61/00*(2006.01)n, *C07C27/00*(2006.01)n, *C07C51/235* (2006.01)n, *C07C57/04*(2006.01)n
According to International Patent Classification (IPC) or to both national classification and IPC

B. FIELDS SEARCHED

Minimum documentation searched (classification system followed by classification symbols)
B01J23/30, B01J23/20, C07C45/38, C07C47/22, C07B61/00, C07C27/00, C07C51/235, C07C57/04

Documentation searched other than minimum documentation to the extent that such documents are included in the fields searched
Jitsuyo Shinan Koho          1922-1996     Jitsuyo Shinan Toroku Koho     1996-2011
Kokai Jitsuyo Shinan Koho    1971-2011     Toroku Jitsuyo Shinan Koho     1994-2011

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | WO 2009/136537 A1  (Nippon Kayaku Co., Ltd.), 12 November 2009 (12.11.2009), example 19 & US 2011/0112330 A       & EP 2275204 A1 & CN 102066001 A        & KR 10-2011-0011603 A | 1-7 |
| A | JP 2007-137785 A  (Nippon Shokubai Co., Ltd.), 07 June 2007 (07.06.2007), paragraphs [0021], [0029]; table 1; paragraphs [0038], [0039] & WO 2007/058221 A1 | 1-7 |
| A | CHAI Song-hai, et al., Sustainable production of acrolein:Gas-phase dehydration of glycerol over $Nb_2O_5$ catalyst, Journal of Catalysis, 2007. 09.10, Vol.250, No.2, page.342-349 | 1-7 |

☒ Further documents are listed in the continuation of Box C.      ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11 October, 2011 (11.10.11) | 25 October, 2011 (25.10.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

## INTERNATIONAL SEARCH REPORT

| | International application No. |
|---|---|
| | PCT/JP2011/065669 |

C (Continuation).  DOCUMENTS CONSIDERED TO BE RELEVANT

| Category* | Citation of document, with indication, where appropriate, of the relevant passages | Relevant to claim No. |
|---|---|---|
| P,A | JP 2010-155183 A  (Nippon Shokubai Co., Ltd.), 15 July 2010 (15.07.2010), claims; examples (Family: none) | 1-7 |

Form PCT/ISA/210 (continuation of second sheet) (July 2009)

**REFERENCES CITED IN THE DESCRIPTION**

**Patent documents cited in the description**

- JP 2010099596 A
- JP 2006290815 A
- JP 6211724 A
- WO 2006087083 A
- WO 2006087084 A
- WO 2007058221 A
- JP 3845720 B

**Non-patent literature cited in the description**

- **S.H.CHAI et al.** *Journal of Catalysis,* 2007, vol. 250, 342-349